# FASCICULE DE BREVET EUROPEEN

(11) **EP 1 876 974 B1**
(45) Date de publication et mention de la délivrance du brevet: **25.07.2012**
(21) Numéro de dépôt: 06721562.4
(22) Date de dépôt: 08.05.2006
(51) Int. Cl.: A61B 17/15, A61G 13/00

(54) **INSTRUMENT DE VISÉE POUR LA DÉTERMINATION DE L'AXE MÉCANIQUE DU FÉMUR**
ZIELINSTRUMENT ZUR BESTIMMUNG DER MECHANISCHEN ACHSE DES FEMURS
SIGHTING INSTRUMENT FOR DETERMINING THE MECHANICAL AXIS OF THE FEMUR

(30) Priorité: 06.05.2005 FR 0504607
(43) Date de publication de la demande: 16.01.2008
(73) Titulaire: Collette, Michel, 1180 Bruxelles (BE)
(72) Inventeur: Collette, Michel, 1180 Bruxelles (BE)
(74) Mandataire: Colens, Alain M.G.M.
(86) Numéro de dépôt international: PCT/BE2006/000049
(87) Numéro de publication internationale: WO 2006/119591

(56) Documents cités:
- FR-A- 2 829 376
- US-A- 5 007 912
- US-A- 5 520 694
- US-A- 5 611 353
- US-B1- 6 554 837

## Description

La présente invention concerne notamment un appareil et un procédé pour aider à déterminer ou estimer l'orientation de l'axe mécanique du fémur.

Il est en effet bien connu qu'une prothèse de genou doit être placée perpendiculairement à l'axe mécanique du membre inférieur. Cet axe passe par le centre de rotation de la hanche, le centre du genou et le centre de la cheville. Une arthroplastie du genou exige donc la réalisation de coupes osseuses au niveau de la partie distale du fémur avec une orientation la plus précise possible.

### ART ANTERIEUR

La méthode classique pour réaliser cet objectif consiste à mesurer sur une grande radiographie préopératoire du membre inférieur l'angle (alpha) entre l'axe diaphysaire (a) du fémur (réel) et l'axe mécanique (M) (virtuel). Lors de l'intervention, on utilise l'axe diaphysaire fémoral pour y introduire une tige guide permettant d'enfiler les instruments de coupe avec un angle égal à l'angle alpha mesuré sur la radiographie préopératoire.
Plus récemment, la navigation informatique a permis de modéliser le fémur opéré et de visualiser virtuellement son axe mécanique de manière à ajuster le placement de la prothèse selon un plan aussi proche que possible du plan perpendiculaire à cet axe.

La première méthode, traditionnellement utilisée depuis de nombreuses années manque malheureusement de précision. En effet, il peut y avoir des erreurs de mesure d'angle sur le cliché préopératoire (erreurs de rotation etc.). Il peut y avoir également plusieurs degrés d'imprécision lors de l'introduction de la tige métallique dans la diaphyse fémorale pour peu que cette diaphyse soit relativement large et autorise une certaine liberté au trajet de la tige. Il n'est donc pas rare de constater sur les clichés postopératoires qu'une prothèse a été placée avec plus de trois degrés d'écart par rapport à l'axe idéal. En outre, l'introduction de la tige dans la diaphyse fémorale n'est pas sans inconvénient puisqu'elle détruit dans une certaine mesure la moelle osseuse diaphysaire et que des embolies graisseuses ont été décrites en postopératoire possiblement liée à l'introduction de cette tige.

La deuxième méthode, dite de navigation informatique,paraît plus précise et les études d'efficacité de cette méthode démontrent clairement qu'elle permet d'obtenir une réduction significative du degré d'erreur de positionnement de la prothèse par rapport à l'axe mécanique.

Cependant, cette méthode nécessite d'une part le déploiement de moyens techniques sophistiqués et coûteux et d'autre part, la mise en oeuvre de cette méthode entraîne généralement une augmentation du temps opératoire généralement évaluée entre 20 et 30 minutes.

De manière plus spécifique, l'art antérieur peut être représenté notamment par les documents suivants :

| | |
|---|---|
| - FR 2 829 376 | Bergue Bertrand |
| - US 5 690 638 | Dance et al |
| - WO 2004/041097 | Aesculap AG & Co. KG |
| - WO 02/47559 | Aesculap AG & Co. KG |
| - EP 1 421 042 | Zimmer Technology, Inc. |
| - EP 0 839 501 | Osteonics Corp |
| - EP 0 677 274 | Osteonics Corp |
| -US5611353 | Osteonics Corp |

Dans ce dernier document il est décrit une méthode dans laquelle on détermine au moins trois points de l'espace correspondant à trois positions du genou et un ordinateur calcule et déduit la position du centre de la tête fémorale.

La présente invention permet de connaître de façon simple rapide et peu coûteuse la localisation exacte de l'axe mécanique par rapport au genou et par conséquent d'ajuster avec une grande précision le positionnement de la prothèse par rapport à cet axe. Elle permet par ailleurs d'éviter de devoir placer une tige métallique à l'intérieur de la cavité médullaire fémorale.

### EXPOSE GENERAL DE L'INVENTION

Selon l'invention on propose de manière générale un appareil pour aider à la détermination de l'axe mécanique du fémur ou de la direction du centre de la tête fémorale par rapport au centre du genou.

Selon un premier aspect de l'invention, on propose un appareil pour la détermination d'un plan contenant l'axe mécanique du fémur comprenant :
- un moyen mécanique d'éléments articulés et/ou coulissants pour la détermination et la mémorisation de deux positions dans l'espace (F1 et F2) d'un même point quelconque (F) du genou lorsque celui-ci se trouve en position P1 et P2, par rapport à un référentiel configuré pour être fixe par rapport au bassin du patient, les dites positions étant obtenues par pivotement du membre inférieur depuis la position P1 à la position P2, autour du centre de la tête fémorale,
- un moyen mécanique pour matérialiser l'orientation d'un plan (oméga) passant par le milieu du segment de droite défini par les deux dites positions F1 et F2, perpendiculairement à ce segment et contenant le centre de la tête fémorale.

Les moyens de repérage peuvent être des éléments cylindriques, par exemple creux, aptes à se positionner avec précision au dessus d'un repère fixé au point F du genou. Ces moyens de repérage peuvent être des guides de percage pour insérer ou enlever une broche servant de repère.

Outre une broche, d'autres types de repère peuvent être proposés par exemple par marquage au laser ou par utilisation d'un crayon ou marqueur chirurgical.

Le moyen pour matérialiser l'orientation est de préférence un moyen mécanique permettant d'orienter une tige au milieu de, et perpendiculairement, au segment de droite séparant les deux dits moyens de répérage.

On peut également avantageusement prévoir un moyen permettant de désolidariser ladite tige pour, par exemple, la reporter et la fixer sur le fémur.

L'appareil peut être conçu pour permettre ledit pivotement dans un plan frontal, dans un plan sagittal ou de préférence dans les deux plans.

L'appareil est fixé à un référentiel qui peut être par exemple une table d'opération ou le bassin du patient, par exemple au niveau de l'os iliaque, plus particulièrement de la crête iliaque.

On comprendra que d'autres variantes sont possibles, pour agencer des articulations et coulissements pour aboutir à un résultat similaire.

Ainsi selon encore une autre variante, un système à quatre tiges articulées formant un parralélogramme, du type pantographe, peut être avantageusement utilisé. Un des sommets du parallélogramme, solidaire d'une structure de support à la table d'opération ou au bassin du patient, est immobilisé par rapport au référentiel choisi et détermine, à l'aide d'un moyen de repérage, le point F1 à la position P1 du genou. Le sommet opposé peut être librement amené par étirement face à la position P2 pour déterminer F2 par un deuxième moyen de repérage. Une tige mécaniquement solidarisée et passant par les deux autres sommets indiquera la direction du centre de la tête fémorale. Dans ce système on ne doit plus ajuster la position du membre inférieur à la position imposée par l'instrument. C'est au contraire l'instrument qui va chercher la position de F2 par déplacement d'un sommet du pantographe jusqu'à ce qu'il coincide avec la position dans l'espace occupée par le repère osseux.

Selon encore une autre variante, on peut concevoir une barre, sous la forme d'un bras, de préférence une barre graduée, fixe, perpendiculaire ou non à l'axe de la table d'opération, ou pivotante, comprenant un curseur muni d'un moyen de repérage, qui est actionné pour coulisser à l'extrémité du bras et déterminer le point F2 en position P2 du genou. Le curseur se déplace le long d'une section d'extrémité distale du bras comportant de préférence une fente laissant passer le moyen de repérage. Le second moyen de repérage est fixé sur la barre pour déterminer le point F1 en position P1, au niveau ou non du pivot éventuel. Un deuxième curseur peut coulisser sur la barre et être amenée au centre des deux moyens de repérage immobilisés de manière connue après la détection de F1 et F2. En appliquant le même principe on peut faire appel à un système de barres télescopiques, la barre coulissante comprenant à son extrémité le moyen de repérage pour déterminer F2.

### LISTE DES FIGURES

L'invention sera davantage comprise à la lecture de la description qui suit, à titre d'exemple uniquement, en se référant aux dessins en annexe dans lesquels :
- la fig. 1 illustre de manière générale l'axe mécanique du membre inférieur,
- les figs. 2 à 6 décrivent le principe géométrique à la base de l'invention

- la fig. 7 décrit le support fixe d'un appareil selon l'invention
- la fig. 8 décrit la partie mobile formant le système de visée
- la fig. 9 montre du dessus un ensemble de l'appareil montré
- la fig. 10 est une coupe verticale illustrant un bras avec un axe creux
- la fig. 11 est une coupe de l'axe avec un tube porte broche traversant la partie creuse
- la fig. 12 décrit le support formant canon de visée fixe et mobile
- les figs. 13 à 14 décrivent le positionnement de l'appareil par rapport au genou

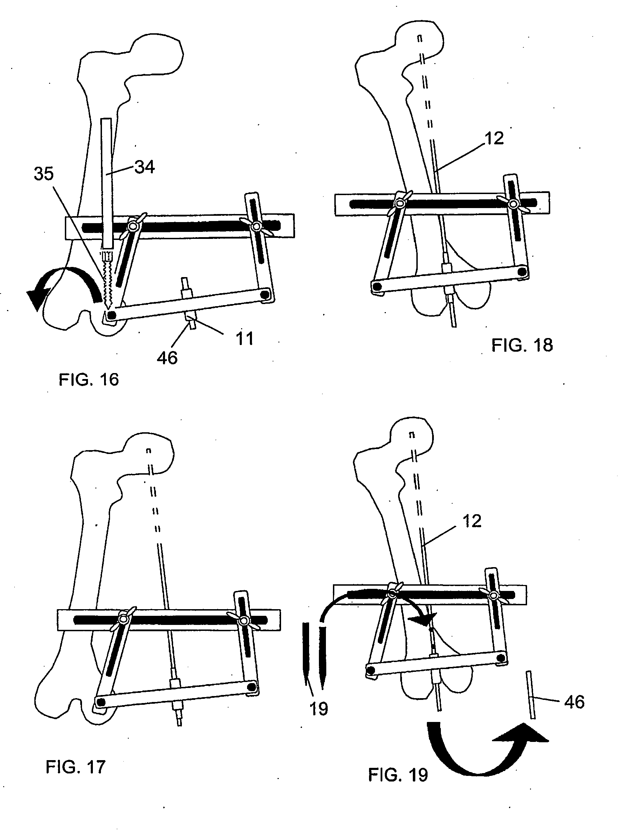

- les figs. 15 à 19 décrivent les étapes d'utilisation de l'appareil selon l'invention
- la fig. 20 montre un fémur muni de la tige déterminant l'axe mécanique du membre inférieur
- les figs. 21 à 26 décrivent un autre mode de réalisation de l'invention, actuellement moins préféré.
   Les figs 27 à 42 décrivent le mode de réalisation actuellement préféré de l'invention, dans lesquels

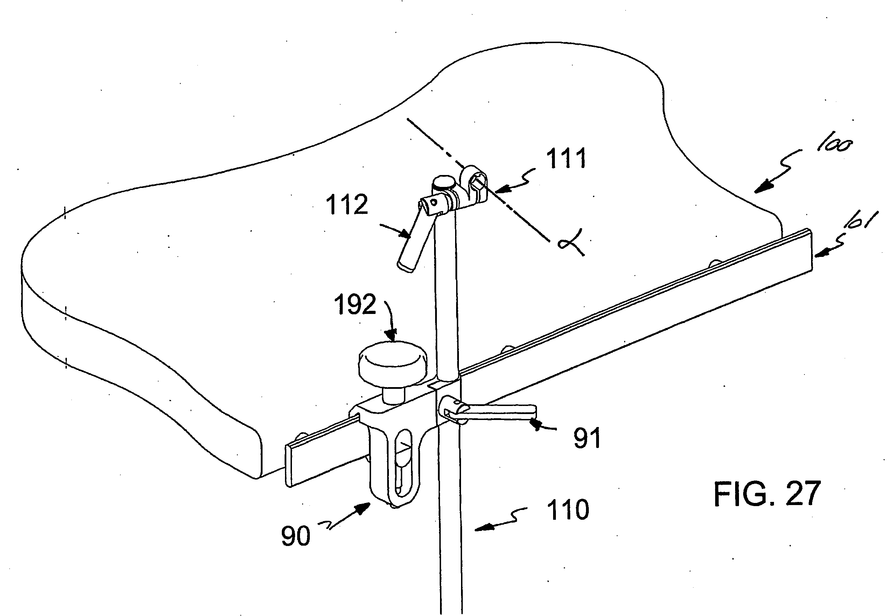

- la fig. 27 illustre en perspective un premier ensemble d'éléments articulés fixé à un une table d'opération

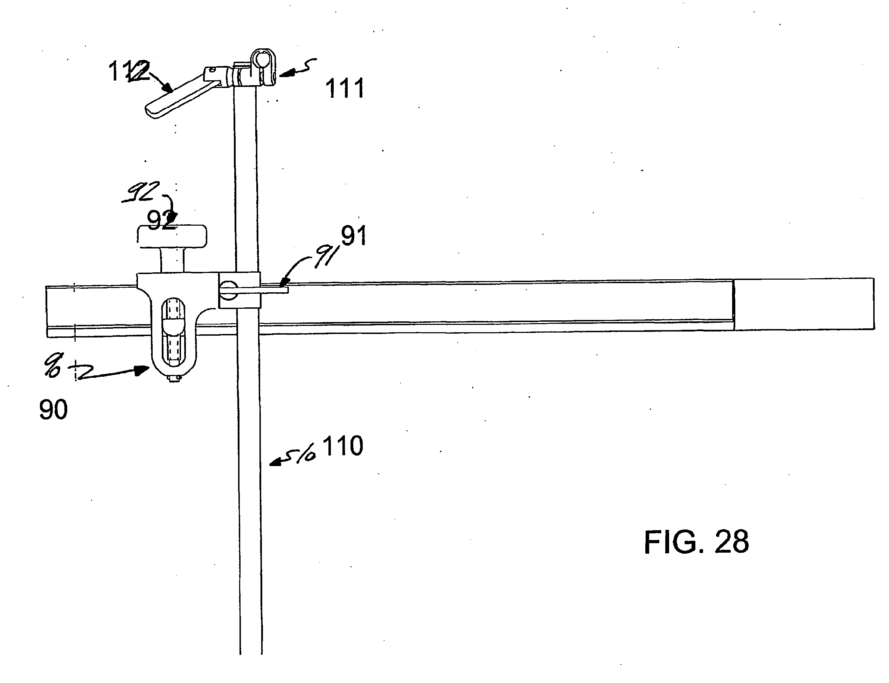

- la fig. 28 illustre le même ensemble en vue latérale

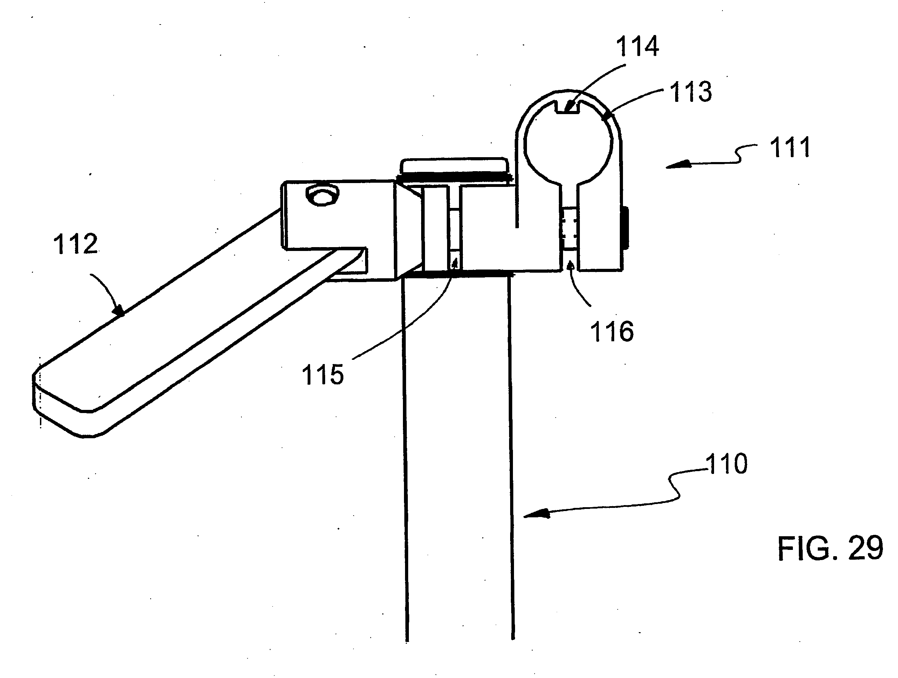

- la fig. 29 illustre en détail une molette de cet ensemble

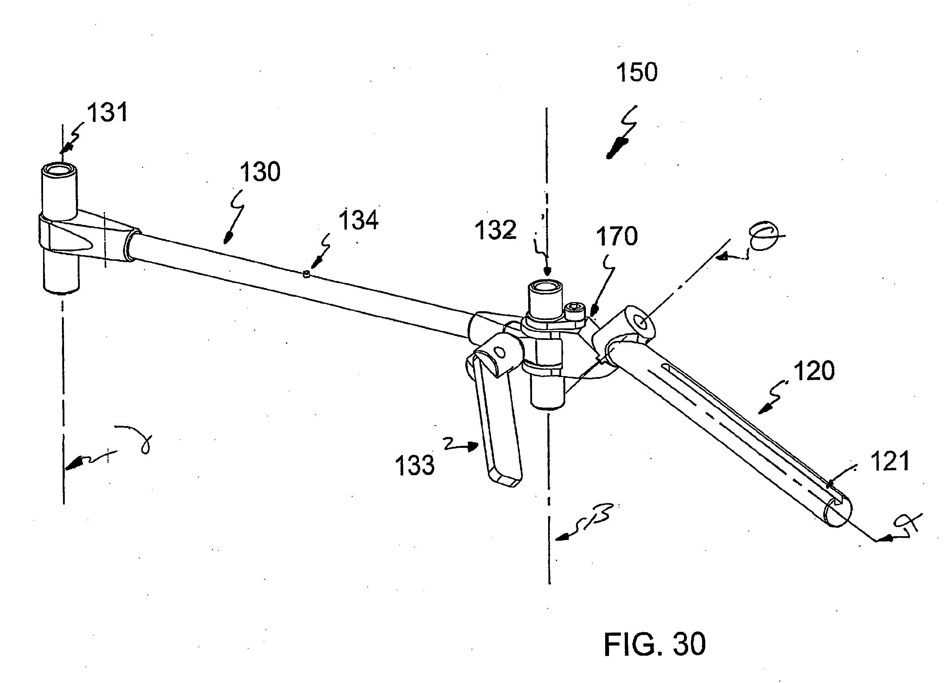

- la fig. 30 illustre en perspective un deuxième ensemble d'éléments articulés destiné à coopérer avec le premier ensemble

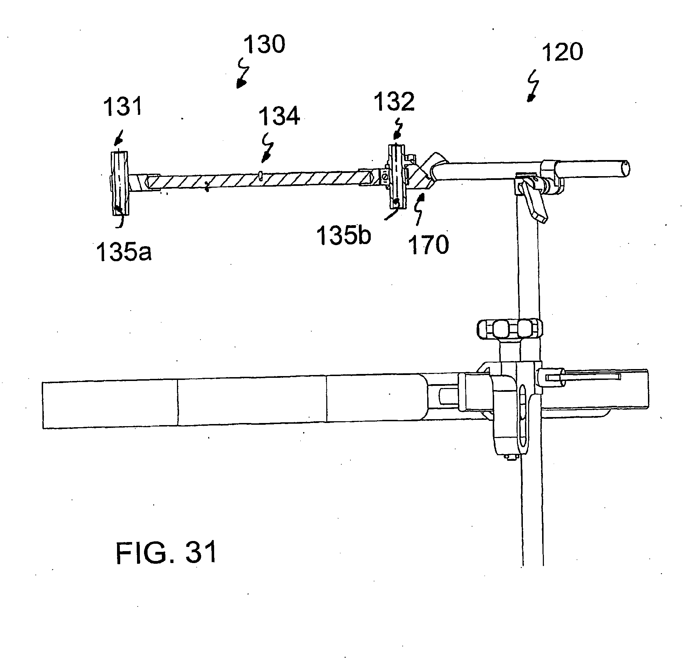

- la fig. 31 est une coupe verticale passant par le centre d'une barre de réglage final du second ensemble

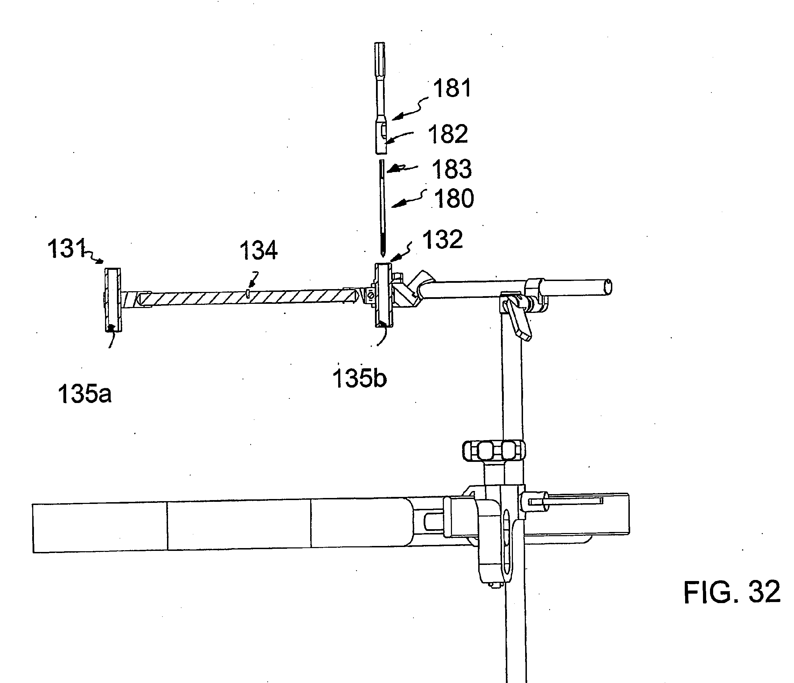

- la fig. 32 illustre un embout et une broche filetée coopérant avec un guide prévu dans ladite barre de réglage final

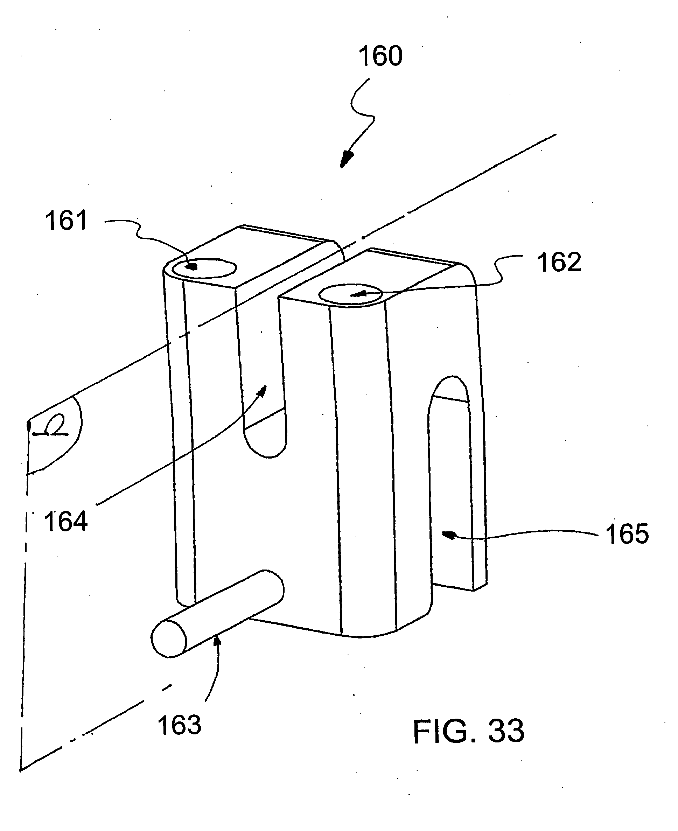

- la fig. 33 illustre un bloc de visée fixé sur ladite barre de réglage final

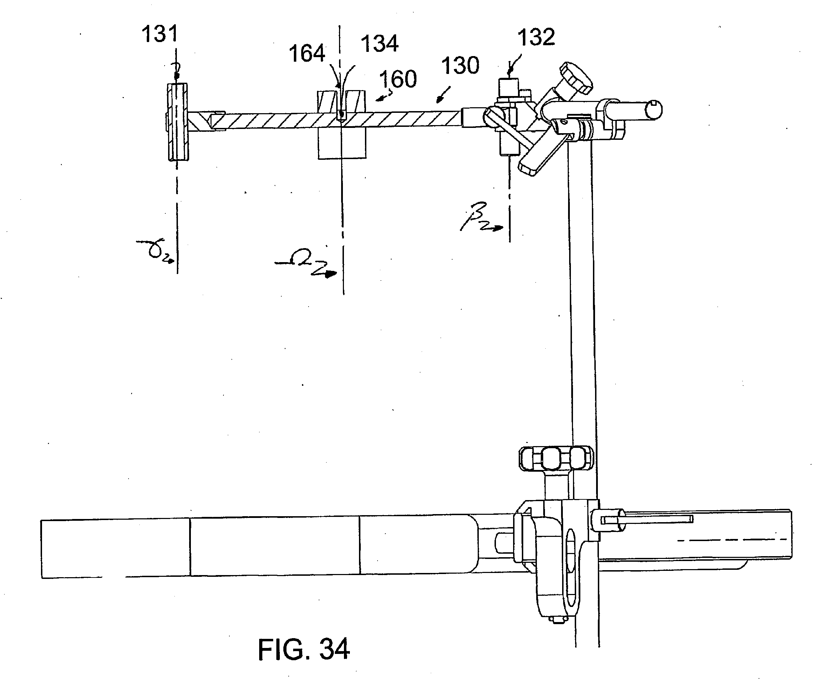

- la fig. 34 est une coupe explicitant davantage la fig. 33

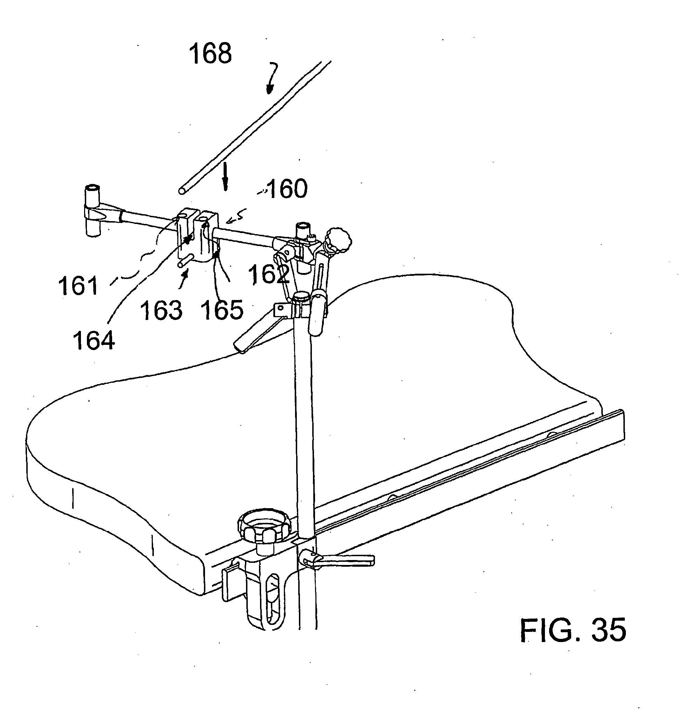

- la fig. 35 montre la mise en place d'une tige ou barre d'alignement coopérant avec le système de visée

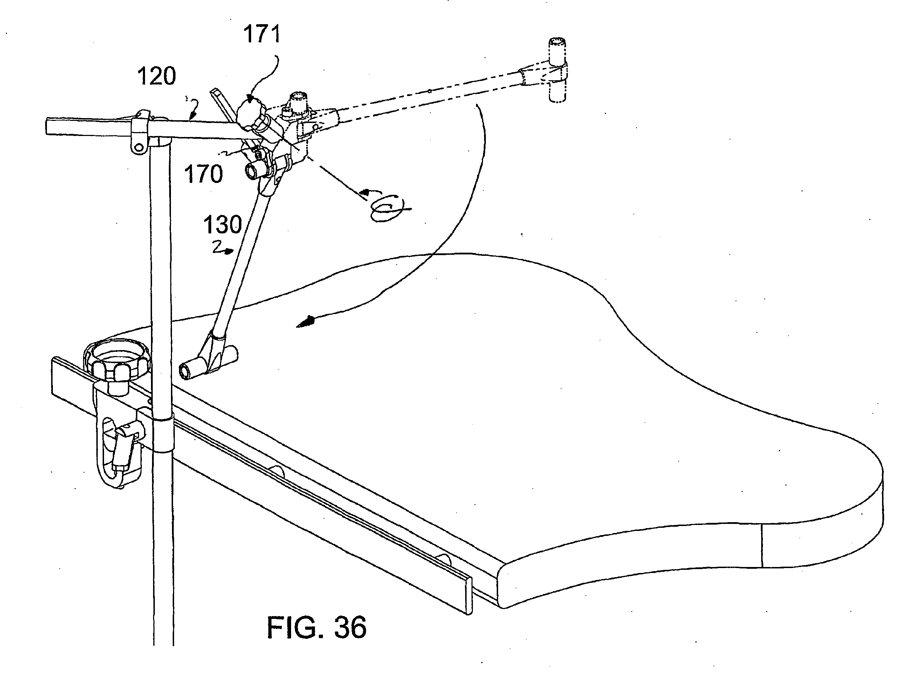

- la fig. 36 explique le changement de configuration de l'instrument permettant de passer d'une mesure dans le plan frontal à une mesure dans le plan sagittal,

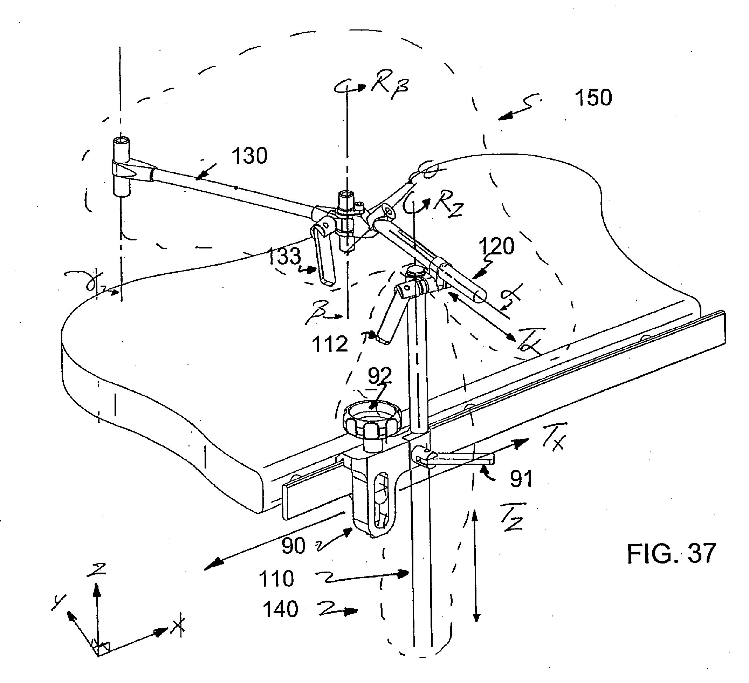

- la fig. 37 est une vue d'ensemble des deux ensembles d'éléments articulés couplés fonctionnellement avec les différents degrés de liberté indiqués,

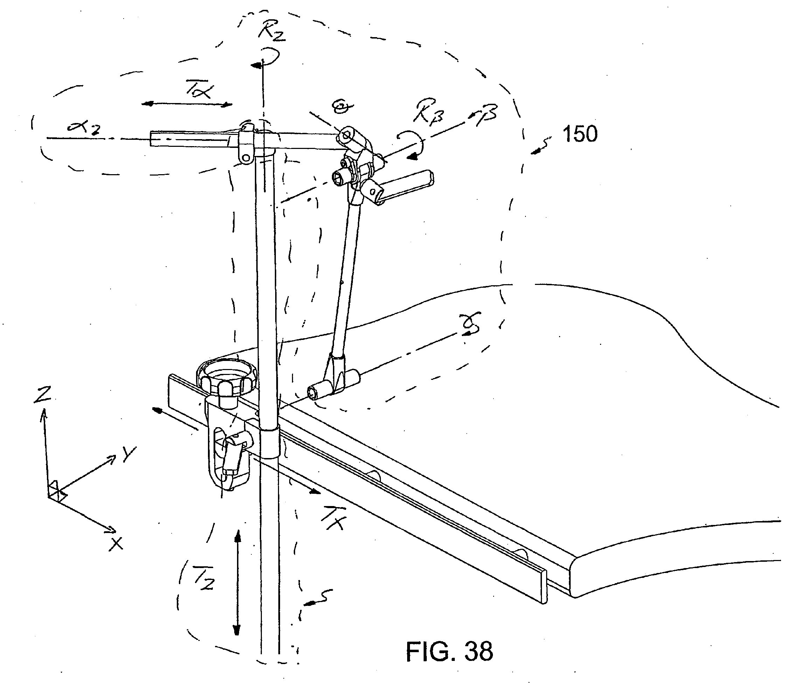

- la fig. 38 est une vue correspondante pour les réglages dans le plan sagittal,

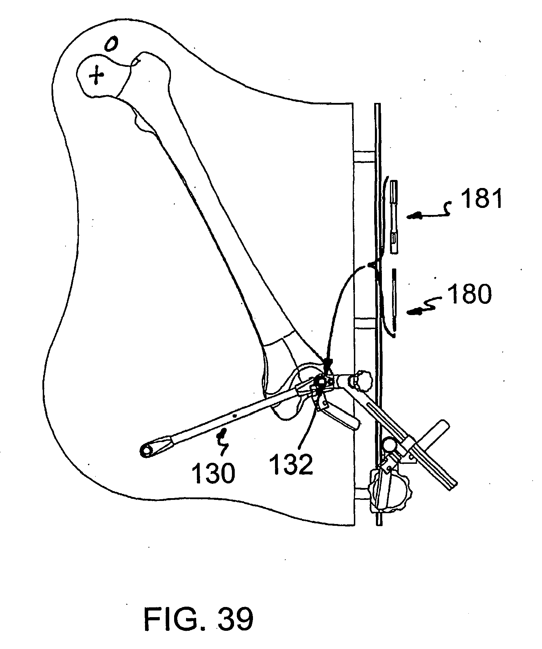

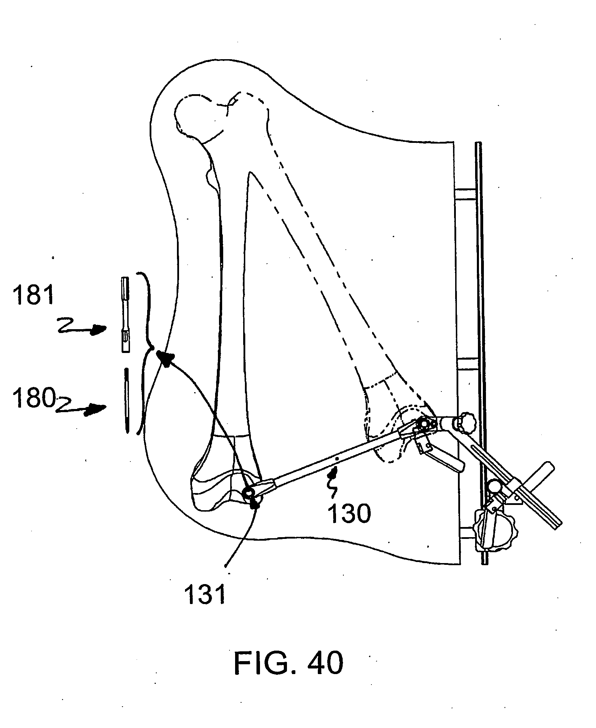

- les figs. 39 à 42 illustrent la technique d'utilisation de l'instrument,

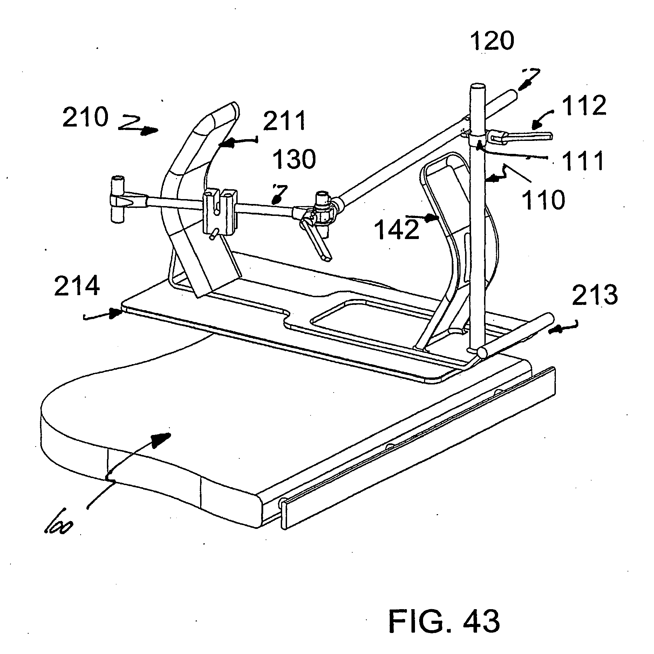

- la fig. 43 concerne une variante du mode de réalisation préféré dans laquelle le référentiel est le bassin du patient et non plus la table d'opération,

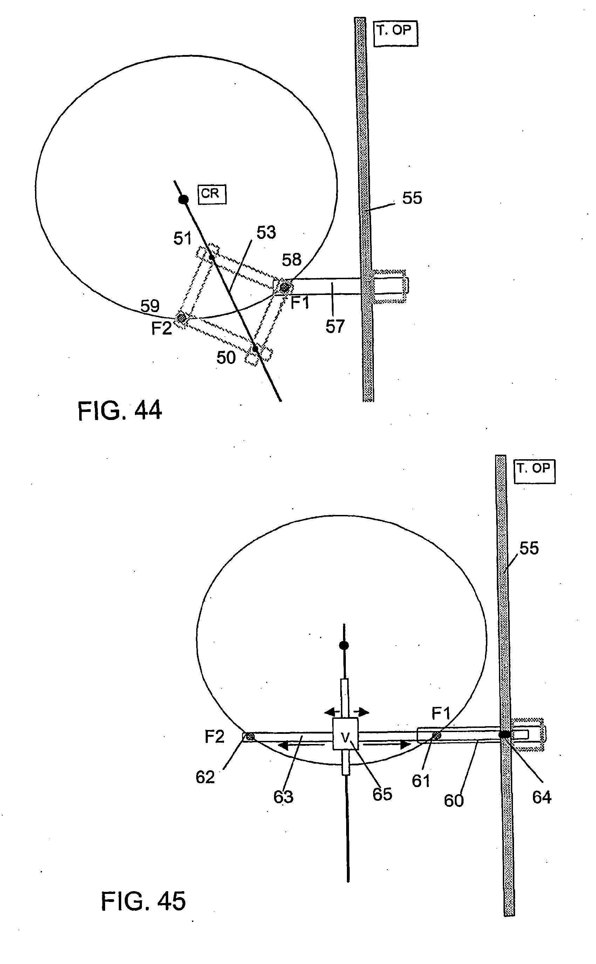

- la fig. 44 illustre de manière schématique une variante de l'invention particulièrement avantageuse faisant appel à un quadrilatère articulé du type pantographe
- la fig. 45 illustre également de manière schématique encore une autre variante de l'invention faisant appel à un organe télescopique.

### DISCUSSION DES FIGURES

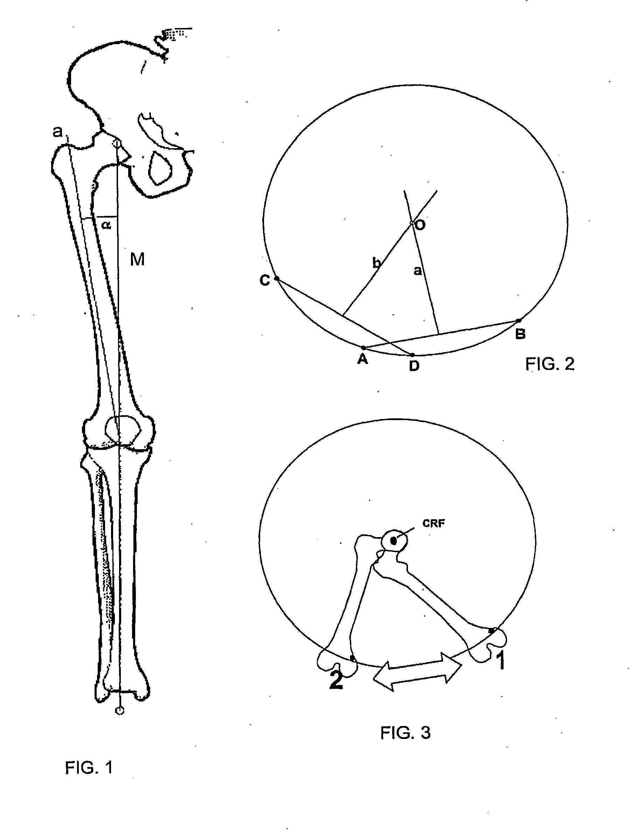

Les figs. 2 à 6 expliquent le principe géométrique à la base de l'invention.

La perpendiculaire abaissée au milieu du segment de droite joignant deux points quelconques d'un cercle géométrique passe par le centre de ce cercle O (Fig 2).

Par exemple, la droite perpendiculaire "a" abaissée au milieu de AB passe par le centre O du cercle auquel appartiennent les points A et B.
La perpendiculaire "b" abaissée au milieu de la droite joignant deux autres points quelconques (C et D) du même cercle passe également par le centre O

Lorsque le fémur pivote dans le plan frontal de la position 1 (P1) à la position 2 (P2), tout point appartenant au fémur tourne autour de son centre de rotation CRF qui passe par le centre de la hanche (Fig 3).

Si l'on marque un point quelconque du fémur, par exemple F, et que l'on garde en "mémoire" la position dans l'espace de F lorsque le fémur est en position 1, on a identifié la position spatiale de F en P1, soit F1. On peut ensuite repérer la position du même point F du genou lorsque le fémur est en position 2 et identifier la localisation spatiale de F en P2 soit F2.

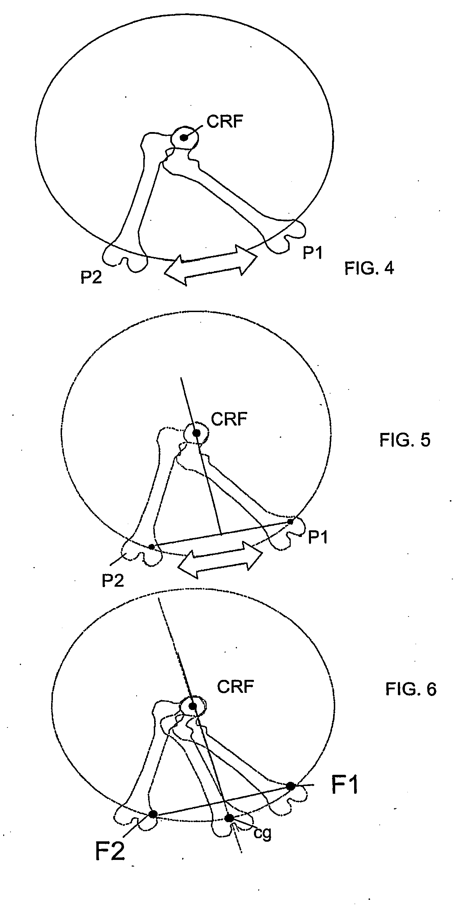

La perpendiculaire passant par le milieu de la droite joignant F1 et F2 dans un plan contenant le centre de la tête fémorale (plan frontal) passe par définition par le centre de la hanche CRF (fig. 5).

Enfin, si l'on amène le fémur dans une position telle que cette perpendiculaire passe par le centre du genou Cg, on a déterminé l'orientation de l'axe mécanique du fémur qui par définition est la droite joignant le centre de la hanche au milieu du genou (fig. 6).

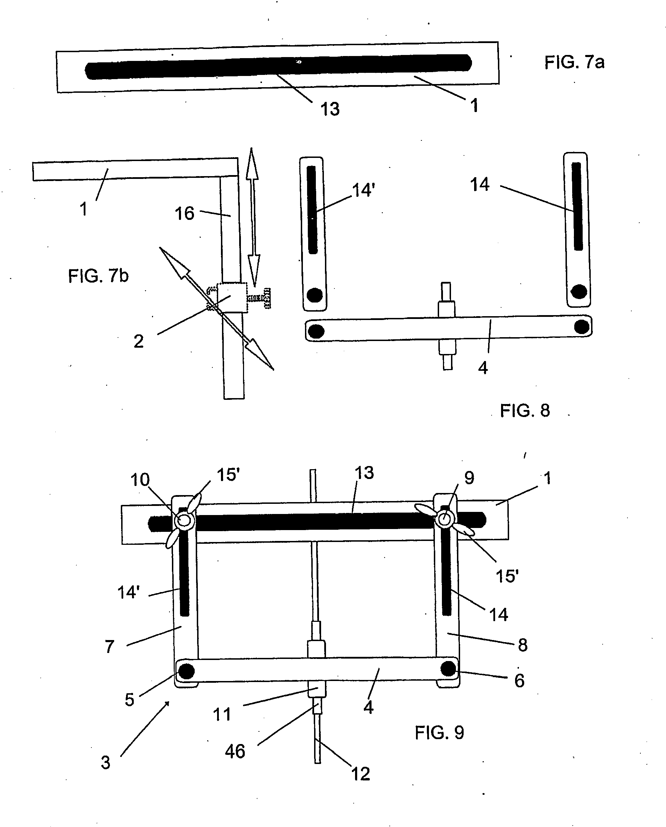

Les figures 7 à 9 décrivent un mode particulier de réalisation de l'invention sous la forme d'une structure de barres articulées formant quadrilatère, composé d'une partie fixe (support fixe, figs. 7a et 7b) et d'une partie mobile (fig. 8) ou système de visée.

Les figures 7a et 7b illustrent le profilé de la partie fixe vu respectivement d'en haut et latéralement.

Il s'agit d'une barre rigide en forme de L comprenant un segment vertical 16 et un segment horizontal 1. Le segment vertical 16 passe dans un étau 2 qui permet de régler la position du support par rapport au malade. L'étau 2 coulisse sur un rail métallique (non illustré) fixé le long de toute table d'opération. Ce dispositif permet de régler la position de la barre en L , d'une part horizontalement en direction de la tête ou des pieds du malade selon l'axe longitudinal de la table d'opération et , d'autre part, verticalement en direction du plafond ou du sol de la salle d'opération.

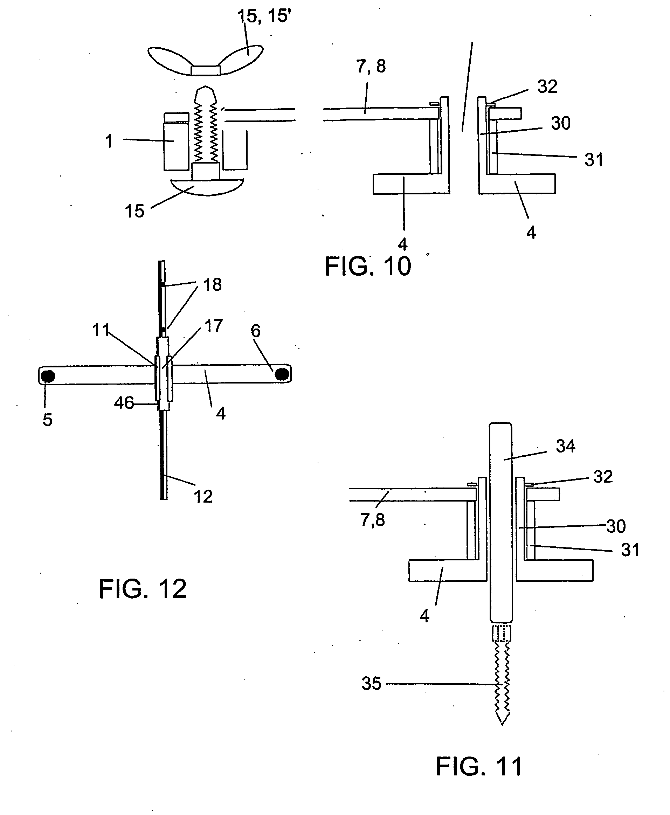

La barre horizontale 1 est fendue sur toute sa longueur afin de permettre l'introduction et le coulissement des boulons 15 de fixation du système de visée (figs. 9, 10) articulé.

La partie mobile ou système de visée (fig. 8) comprend deux bras mobiles 7, 8 et une plaque transversale 4 qui porte l'organe de visée proprement dit , qui se compose du canon ou support fixe 11 et du canon mobile 46 .

Les bras mobiles 7 et 8 (fig 8) se composent chacune d'une plaque métallique d'environ 15 cm de long sur 25 mm de large et 2 à 3 mm d'épaisseur. Chaque bras est percé d'une fente 14 longitudinale de 8 à 10 cm de long sur 5 à 6 mm de large (en fonction du calibre des boulons de fixation) destinée à permettre l'introduction et le coulissement du boulon 15 de fixation solidarisant le bras 7, 8 à la barre horizontale 1 du support fixe.

L'autre extrémité de la plaque est percée d'un orifice circulaire destiné à s'articuler avec le pivot cylindrique situé à chaque extrémité de la plaque transversale 4, créant ainsi entre les deux éléments un mouvement rotatoire pur autour de l'axe 5 ou 6 passant par le centre du pivot cylindrique 30 (fig. 10).

La figure 9 montre une vue en plan de dessus du système de visée articulé avec la barre horizontale 1 du support fixe.

La figure 10 montre une coupe verticale passant par le centre du bras mobile ( 7 ou 8) selon son axe longitudinal.

La plaque transversale 4 (figs 8, 9, 10) se compose d'une plaque métallique d'environ 20 cm de long sur 25 mm de large et d'environ 3 mm d'épaisseur. Sur sa face inférieure est fixé le canon de visée 11 proprement dit déjà mentionné.

Chacune des extrémités supérieures de la plaque est munie d'un pivot cylindrique creux 30 de 2 à 3 cm de haut autour duquel s'articule le bras mobile 7, 8 par l'intermédiaire de l'orifice circulaire situé à son extrémité (fig. 10).Entre ces deux éléments se trouve une bague cylindrique d'écartement 31 coulissant autour de chaque pivot cylindrique de la plaque transversale et maintenant l'écartement entre le bras mobile et la plaque transversale. Cette bague 31 a pour effet d'abaisser le niveau de la plaque transversale 4 par rapport au bras mobile 7, 8 et à la barre horizontale 1 du support fixe de manière à ce que la tige de visée 12 de l'axe puisse passer librement en dessous d'elle.

Après articulation des éléments, un système d'arrêt (rivet 32 ou autre dispositif équivalent) fixé sur l'extrémité du pivot cylindrique 30 empêche les pièces de se désolidariser tout en permettant le mouvement rotatoire de l'une par rapport à l'autre.

L'orifice intérieur du pivot cylindrique 30 sert de guide à l'introduction du tube cylindrique 34 portant la broche filetée 35 qui sera introduite dans le genou (fig. 11) au point F. Le diamètre intérieur du pivot cylindrique de la plaque sera donc très légèrement supérieur au diamètre extérieur du porte-broche.

La figure 12 montre une vue du dessous de la plaque transversale avec son canon de visée. Celui ci comprend un canon fixe 11 dans lequel coulisse un canon mobile 46 qui guide la tige de visée 12.

Le canon fixe 11 est un cylindre métallique d'environ 3 cm de long fixé sur la face inférieure de la plaque transversale 4.
L'axe longitudinal de ce canon est strictement perpendiculaire au grand axe de la plaque transversale 4 et se trouve exactement au milieu de la distance qui sépare l'axe central de chaque pivot cylindrique 5, 6.
Il est percé d'une fente longitudinale 17, orientée vers le bas dont la largeur est insuffisante pour laisser passer le canon mobile 46 mais suffisante pour laisser passer la tige de visée 12.

Le canon mobile 46 est un cylindre de 5 à 6 cm de long dont le diamètre extérieur est ajusté pour coulisser étroitement mais librement dans le canon fixe 11.

La tige de visée 12 est une tige d'environ 20 cm qui coulisse dans le canon mobile 16. Elle est percée de plusieurs petits orifices 18 permettant l'introduction de petites broches métalliques destinées à fixer la tige guide au fémur après que son orientation selon l'axe mécanique recherché ait été déterminée.

Son diamètre étant inférieur à la largeur de la fente 17 pratiquée dans le canon fixe 11, il suffit pour la dégager d'enlever le canon mobile 46 par coulissement et tout l'appareil peut alors être ôté en bloc vers le haut en déserrant l'étau de fixation 2.

### Technique d'utilisation de l'instrument

Le patient est installé classiquement en décubitus dorsal. On s'assurera que le rail de fixation des accessoires de table d'opération est suffisamment saillant pour être palpé à travers les champs stériles après leur mise en place. Dans le cas contraire un segment de rail intermédiaire supplémentaire peut être fixé sur le rail original de manière à ce que cette pièce soit facilement palpée lorsque les champs opératoires auront été mis en place, afin d'y fixer facilement l'étau stérile portant le support fixe de l'instrument.

Après mise en place des champs stériles, le genou est abordé selon la technique habituelle.

L'articulation étant exposée , on met en place l'étau stérile 2 qui se fixe par dessus les champs opératoires (en les renforçant si nécessaire à l'endroit de fixation par un petit champ plastique autocollant).

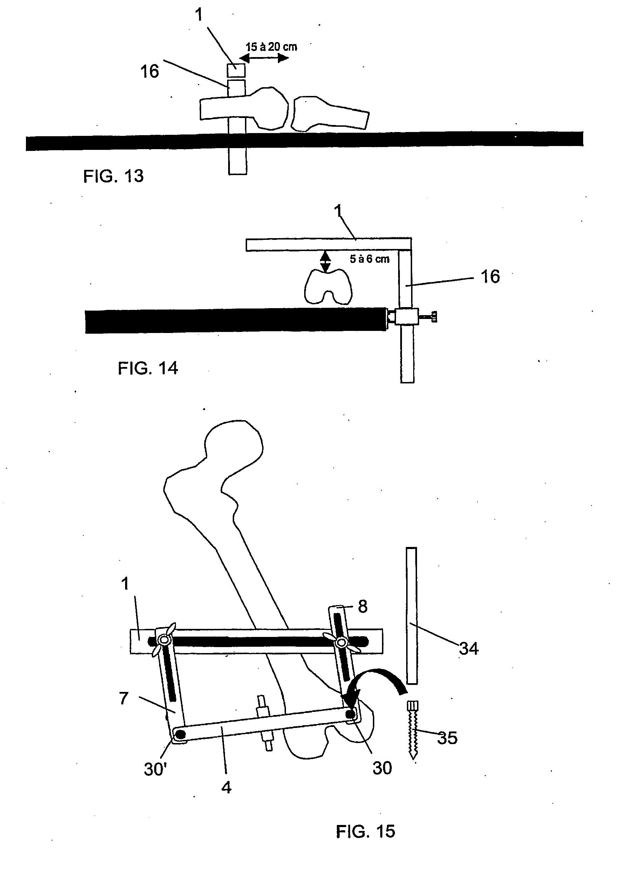

La position de l'étau 2 est réglée de telle manière que la barre horizontale 1 surplombe le genou G du patient environ 15 cm cranialement (en direction de la tête) par rapport à l'interligne articulaire (plan horizontal) (fig 13) et 5 à 6 cm au-dessus du genou dans le plan vertical.(fig 14)

Au départ, les deux écrous papillons 15,15' (fig. 9) sont déserrés et les bras mobiles 7, 8 sont donc essentiellement libre en pivotement et translation.

Le genou est amené en dessous d'un des deux pivots cylindriques 30 de la barre transversale 4 et la broche filetée 35 est introduite (au moteur) perpendiculairement dans le genou G grâce au porte broche 34 lui-même guidé par le pivot cylindrique perpendiculairement au plan horizontal du bras mobile 7 ou 8.

Le choix du site d'insertion de la broche filetée est tout à fait aléatoire mais une fois la localisation choisie, il faut impérativement garder la "mémoire" de cette localisation.

Il suffit pour cela de serrer l'écrou papillon 15 du bras mobile 14 ayant servi à l'introduction de la broche 35 avant de retirer le porte broche 34.

Le serrage de cet écrou supprime en effet toute liberté de mouvement du bras 8 dans l'espace ce qui fige en quelque sorte sa position relative par rapport à F1 et donc fige la position F1 dans l'espace occupé par la broche sur l'arc de cercle lorsque le genou se trouve en position P1.

On amène alors le genou sous le pivot cylindrique du deuxième bras mobile 7 qui à ce moment est toujours entièrement libre ce qui permet d'ajuster aisément sa position dans l'espace.

L'ajustement est correct lorsque le porte broche 34 guidé par le pivot cylindrique 30 "retrouve" l'axe de l'empreinte, p.e. hexagonale, de la broche filetée ce qui permet à ce moment de l'extraire du fémur.

Avant le retrait du porte broche, il est impératif de serrer le deuxième écrou papillon 15' de manière à conserver la "mémoire" de la localisation de la broche en position 2 du genou (=F2). La localisation dans l'espace du deuxième point appartenant à l'arc de cercle décrit par la broche lorsque le genou est en position P2 (=F2) est ainsi déterminée.

La tige de visée 12 est alors introduite.

Par construction, le viseur 11 indique l'orientation de la perpendiculaire passant par le milieu du segment de droite joignant les deux points F1 F2 déterminés , indiquant ainsi de façon extrêmement précise la direction du centre de la hanche.

On déplace ensuite le fémur jusqu'à ce que le centre du genou se place exactement dans l'alignement de la tige 12 de visée et on a ainsi matérialisé l'axe mécanique fémoral M.

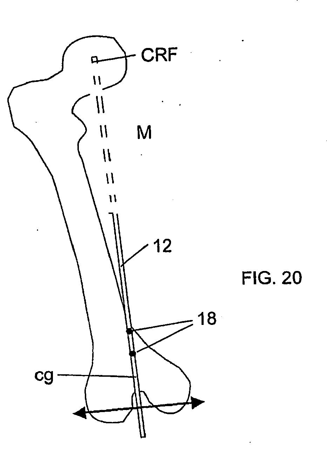

On peut alors fixer la tige 12 au fémur au moyen de petits clous passant par les orifices 18 (fig. 20) prévus le long de la tige (en s'assurant que cette tige reste parallèle au plan frontal).

En enlevant le canon mobile 46 on permet à la tige de s'échapper par la fente longitudinale du canon fixe. L'instrument 3 peut alors être ôté en bloc vers le haut en déserrant simplement l'étau de fixation du support

La tige détermine un plan perpendiculaire au plan frontal et contenant le centre de la tête fémorale. Les instruments de coupe distale fémorale seront orientés perpendiculairement à ce plan ce qui facilitera l'obtention d'un plan de coupe rigoureusement perpendiculaire à l'axe mécanique fémoral. On comprendra cependant que l'orientation dans le plan sagittal reste à être estimé par une technique identique ou complémentaire.

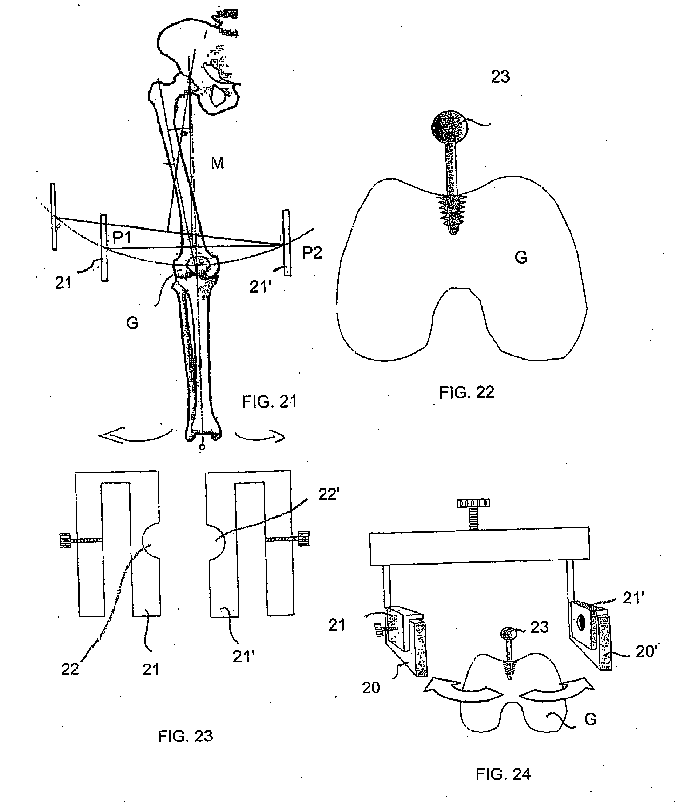

Les figs. 21 à 25 illustrent schématiquement un autre mode de réalisation de l'invention.

Le principe appliqué consiste également à déterminer deux points P1 P2 sur l'arc de cercle de pivotement du membre inférieur dans un plan parallèle au corps. La bissectrice de l'angle formé par cet arc de cercle passe par le centre de rotation et ramenée au genou, détermine l'axe recherché qui va permettre une coupe rigoureusement perpendiculaire de l'os.

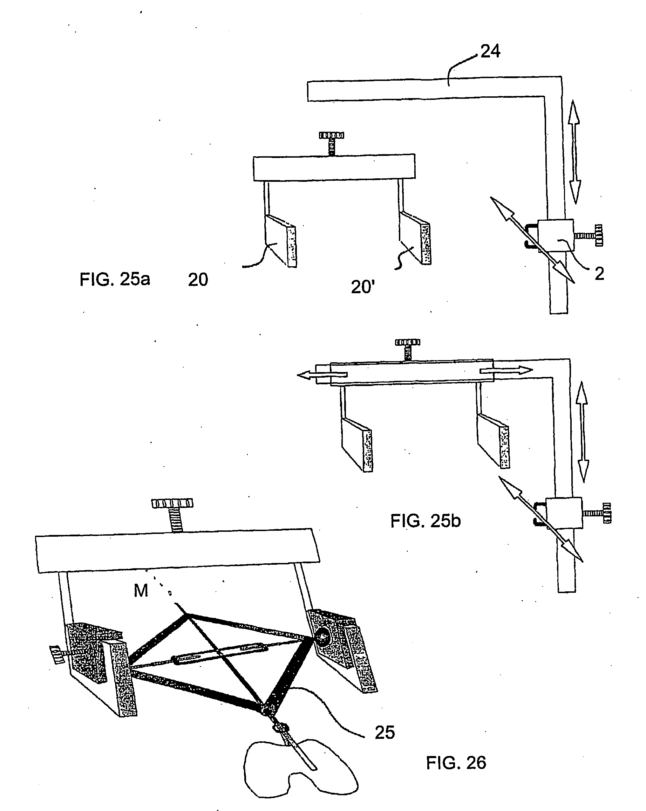

L'instrument est conçu de telle façon que de part et d'autre du genou se trouvent deux plaques 20, 20' parallèles allongées et réglables latéralement sur un support 24 en L (fig. 25) fixé à un référentiel fixe, tel la table d'opération ou le bassin du patient, et pouvant coulisser cranialement et verticalement par exemple par l'intermédiaire d'un étau fixé sur le rail de la table d'opération. Sur ces plaques fixes coulissent un organe cavalier 21, 21' comportant un évidement 22, 22' hémisphérique se faisant face. Ces évidements constituent des récepteurs de contact avec un repère 23 quasi sphérique et solidaire du genou.

L'utilisateur de l'instrument fait pivoter le membre inférieur de façon à ce que le repère fixe 23 solidaire du genou vienne buter sur chacune des plaques 20. La position exacte de P1 et P2 est déterminée en ajustant la position du cavalier 21 de telle sorte que le relief convexe du repère 23 coincide parfaitement avec le relief concave correspondant à l'évidement 22 du cavalier 21, lequel est alors serré sur la plaque 20 pour conserver la mémoire de sa position spatiale.

La bissectrice de l'arc de cercle ainsi déterminé peut être matérialisé par un moyen mécanique tel un pentographe 25 muni d'une tige 12 formant bissectrice et muni de 2 éléments palpateurs sphériques permettant de retrouver avec exactitude la position spatiale des évidements 22 des cavaliers 21.

IL est bien entendu que diverses autres variantes existent pour orienter et immobiliser un bras ou un système articulé avec deux moyens de repérage aptes à s'aligner sur un repère du genou dans deux positions, et ainsi matérialiser, par exemple par une tige perpendiculaire fixée à distance égale desdits deux moyens de repérages, un plan passant par le centre de la tête fémorale.

### DESCRIPTION DETAILLEE D'UN MODE PREFERE DE REALISATION

Les figures 27 à 42 décrivent de manière plus détaillée un mode de réalisation de l'invention sous la forme d'une structure de barres articulées 140 et 150 et d'un système de visée 160.

Pour des raison de simplicité, il est considéré que l'axe principal du corps du patient est aligné avec l'axe principal de la table d'opération 100 et que la table d'opération est strictement horizontale (ou parallèle par rapport au sol). Ainsi le terme vertical signifie perpendiculaire par rapport à la table d'opération et donc par rapport au sol dans le cas présent.

La figure 27 illustre l'ensemble de réglage primaire 140 constitué d'une barre verticale 110 et d'un étau 190 en vue isométrique. Il s'agit d'une barre rigide 110 pouvant coulisser selon l'axe vertical (perpendiculaire par rapport à la table d'opération ou perpendiculaire par rapport au sol considérant la table d'opération strictement horizontale) dans un étau 90 qui permet de régler la position du support par rapport au patient. L'étau 90 dispose d'une molette 91 qui permet de bloquer la translation verticale de la barre 110 à une position déterminée par rapport à l'étau 90. Par ailleurs, l'étau 90 peut translater horizontalement par rapport au rail longitudinal 101 d'une table d'opération 100 destinée à recevoir le patient lors de l'intervention chirurgicale. L'étau 90 dispose d'une molette 92 qui permet de bloquer la translation horizontale de l'étau 90 par rapport à la table 100.

La figure 28 illustre le même ensemble de réglage primaire 140 en vue latérale. La barre rigide 110 peut-être creuse afin de diminuer son poids et peut-être fabriquée de matériaux connus pour la réalisation d'instruments chirurgicaux, tel que l'acier inoxydable mais peut aussi être réalisée à partir de fibre de carbone, ce qui permet de concilier rigidité et légèreté.

A l'extrémité supérieure de la barre rigide 110 se trouve un guide 111 capable de pivoter librement autour de la barre 110. Le guide 111 est caractérise par une ouverture 113 préférablement cylindrique, dont l'axe majeur (alpha) est perpendiculaire par rapport à la barre rigide 110. L'ouverture 113 propose un tenon 114. Une molette 112 permet de bloquer simultanément la rotation du guide 111 par rapport à la barre rigide 110 ainsi que de restreindre l'ouverture 113, ce qui sera mieux compris lors de l'explication de l'utilisation de l'instrument (Figure 29).

La figure 29 montre le.principe de fonctionnent de la molette 112. Le guide 111 agit comme une bride de serrage et propose deux ouvertures 115, 116. Par l'action du vissage de la molette 112, les espaces 115, 116 se referment et bloquent ainsi les degrés de liberté fournis par le guide 111.

La figure 30 illustre l'ensemble de réglage secondaire 150 constitué de deux barres rigides 120 et 130 et d'un renvoi angulaire 170 en vue isométrique. La barre 120 est de section compatible avec l'ouverture 113 du guide 111. Sur une majeure partie de la barre 120 se trouve une fente débouchant 121 à une extrémité de dimension compatible avec le tenon 114. Lors de l'assemblage, le set de réglage secondaire 150 est aligné avec l'ouverture 113 du guide 111. La rotation du set de réglage secondaire 150 par rapport à l'axe majeur (alpha) du guide 111 est limité par le tenon 114 par rapport à la fente 121. A l'extrémité opposée de la fente débouchante, la barre 120 est reliée à un renvoi angulaire 170 qui permet de changer l'orientation de la barre de réglage final 130 du plan frontal au plan sagittal et réciproquement selon le plan de travail decidé.
Le renvoi angulaire 170 intègre un guide de perçage 132. La barre de réglage final 130 peut pivoter autour de l'axe de révolution (Beta) du guide de perçage 132.
Une molette 133 permet de bloquer la rotation de la barre de réglage final 130 par rapport à la barre de réglage 120. A son extrémité opposée, la barre de réglage final 130 intègre un second guide 131 de dimensions similaires au guide 132. L'axe de révolution (gamma) du guide de perçage 131 est parallèle à l'axe de révolution (Bêta) du guide de percage 132. Un téton de localisation 134 est fixé solidairement à la barre de réglage final 130 et se trouve exactement au milieu du segment qui sépare les axes de révolution (Bêta, Gamma des guides 131, 132.

La figure 31 montre la coupe verticale (cv) passant par le centre de la barre de réglage final 130 selon son axe longitudinal. Chacune des extrémités de la barre de réglage final 130 est munie d'un guide de perçage 131, 132 sous la forme d'un pivot cylindrique creux d'une hauteur d'au moins 20 mm.

La figure 32 montre une coupe verticale identique à la figure 31. L'orifice intérieur 135a, 135b des guides de perçage 131, 132 sert de guide à l'introduction de l'embout 181, à portée cylindrique 182, portant la broche filetée 180 qui sera introduite dans le genou comme repère. Il est compris que le diamètre de la portée cylindrique 182 du porte broche 181 est compatible avec le diamètre de l'orifice intérieur 135a, 135b des guides de perçage 131, 132. Par compatible, il doit être compris que le diamètre extérieur de la portée cylindrique 182 du porte broche 181 est légèrement sous-dimensionné par rapport au diamètre de l'orifice intérieur 135a, 135b des guides de perçage 131, 132, mais suffisamment proche pour que les guides de perçage 131, 132 offrent un guidage adéquat du porte broche 182. Par exemple lorsque la broche filetée 180 est introduite dans le genou à travers le guide de perçage 132, son axe d'implantation est confondu avec l'axe Beta du guide de perçage 132.

La figure 33 montre le bloc de visée 160 en vue isométrique. Selon un mode de réalisation, le bloc de visée 160 de forme parallélépipédique dispose d'une première fente 165 débouchante de part et d'autre en forme de U inversée quand le bloc est prêt à être assemblé sur la tige de réglage final 130 et d'une seconde fente 164 débouchante de part et d'autre en forme de U et perpendiculaire à la première fente 165. La seconde fente 164 définit un plan de symétrie Omega, dont de part et d'autre et à distance égale se trouve deux orifices internes débouchants 161, 162 de diamètre similaire et calibrés pour recevoir une broche (non représentée). Les orifices internes débouchants 161, 162 sont à une distance constante de la première fente 165, cela signifie qu'un plan crée par les axes de révolution des deux orifices internes serait parallèle à la première fente 165. La première fente 165 et la seconde 164 sont de longueur suffisante pour déboucher librement l'une dans l'autre. Finalement, au milieu de la face distale parallèle à la première fente 165 se trouve un tenon cylindrique 163 de longueur suffisante pour être aligné avec le centre du genou du patient.

La figure 34 montre la coupe verticale (cv) passant par le centre de la barre de réglage final 130 selon son axe longitudinal et explique le positionnement du bloc de visée par rapport à la barre de réglage finale 130. Il est désormais compris que le bloc de visée 160 se met en place par rapport à la barre de réglage finale 130 en faisant correspondre la fente première 165 avec la barre de réglage finale 130 et en alignant l'ouverture créée par l'intersection des fentes 64, 65 avec le téton de localisation 134. Les dimensions de la première fente 165 sont calibrées avec le diamètre de la barre de réglage final 130 pour permettre le glissement serré du guide 160 par rapport à la barre de réglage final 130, de ce fait le plan de symétrie (omega de la seconde fente est strictement perpendiculaire à la barre de réglage final 130. De plus, le diamètre du téton de localisation 34 est calibré pour correspondre avec l'ouverture créée par l'intersection des fentes 164, 165, de ce fait aucune translation parasite du bloc de visée 160 le long de l'axe principal de la barre de réglage final 130 n'est possible et donc le plan de symétrie (Omega) de la seconde fente 164 se trouve au milieu exact du segment qui sépare les axes de révolution (Bêta, Gamma) des guides de perçage 131, 132.

La figure 35 montre la mise en place d'une barre d'alignement 168 le long de la seconde fente 164. Le diamètre de la barre d'alignement 168 est calibré avec la seconde fente 164 et de ce fait l'axe principal de la barre d'alignement 168 appartient au plan Omega de la seconde fente 164.

La figure 36 explique le changement de configuration de l'instrument pour le passage de la recherche du centre de la tête fémorale dans le plan frontal au plan sagittal. Il existe une vis de blocage 171 qui permet le serrage du renvoi angulaire 170 avec l'extrémité de la barre de réglage 120. Le dévissage de la vis de blocage autour de son axe de rotation (Téta) permet de pivoter le renvoi angulaire 170 et la barre de réglage final 130 par rapport à la barre de réglage 120 et de ce fait de basculer le plan défini par les axes de révolution (Beta, Gamma) de perpendiculaire au plan frontal du patient (représenté par le plan XY) à perpendiculaire au plan sagittal du patient (représenté par le plan XZ). Le passage d'un plan à l'autre obtenu, il suffit de revisser la vis de blocage 171.

La figure 37 explique les réglages disponibles pour ajuster le set de réglage primaire 140 et le set de réglage secondaire 150 dans un référentiel lié à la table d'opération 100 :
- L'étau 90 peut translater le long de l'axe principal de la table 100 : Tx
- La barre rigide peut translater perpendiculairement par rapport à l'étau 90 : Tz
- Le set de réglage secondaire 150 peut translater par rapport à la barre rigide 110 le long de l'ouverture 113 du guide 111 : Talpha.
- Le set de réglage secondaire 150 peut pivoter autour de la barre rigide 110 : Rz
- La barre de réglage final 130 peut pivoter autour de l'axe de révolution (Beta) du guide de perçage 132 : Rbeta.

Après articulation des éléments, des systèmes d'arrêts, modélisés ici par des molettes de serrage 92, 91, 112, 133, empêchent le système de barres 110, 120, 130 et l'étau 90 de se désolidariser.

Dans le cas présent, l'instrument est utilisé pour une recherche du centre de la tête fémorale dans le plan frontal, de ce fait le plan défini par les axes de révolution (Beta, Gamma) est perpendiculaire au plan frontal (représenté par le plan XY).

La figure 38 montre que les réglages disponibles dans le plan sagittal sont identiques à ceux disponibles dans le plan frontal, à la différence que le plan défini par les axes de révolution (Beta, Gamma) est perpendiculaire au plan sagittal du patient (représenté par le plan XZ).

Les figures 13, 20, 39-42 expliquent la technique d'utilisation de l'instrument :
Le patient est installé classiquement en décubitus dorsal. On s'assurera que le rail de fixation des accessoires de la table d'opération est suffisamment saillant pour être palpé a travers les champs stériles après leur mise en place. Dans le cas contraire un segment de rail intermédiaire supplémentaire peut-être fixe sur le rail original de manière à ce que cette pièce soit facilement palpée lorsque les champs opératoires auront été mis en place, afin d'y fixer facilement l'étau stérile 90 portant l'instrument.

Après mise en place des champs stériles, le genou est aborde selon la technique chirurgicale habituelle.

L'articulation étant exposée, on met en place l'étau stérile 90 qui se fixe au rail 101 de la table d'opération 100 par-dessus les champs opératoires (en les renforçant si nécessaire à l'endroit de fixation par un petit champ plastique autocollant).

La position de l'étau 90, la barre rigide 10 et la barre de réglage 120 sont réglées de telle manière que la barre de réglage final 130 (considérant l'instrument configuré pour une recherche du centre de la tête fémorale dans le plan frontal) surplombe le genou G du patient d'environ 15 cm cranialement (en direction de la tête) par rapport à l'interligne articulaire dans le plan horizontal (voir figure 13) et d'environ 5 cm au-dessus du genou dans le plan vertical (voir figure 14).

Au départ, les molettes 92, 91, 112, 133 sont desserrées et donc l'étau 90 peut translater librement le long du rail 101 de la table d'opération 100, la barre rigide 110 peut translater verticalement, le set de réglage secondaire 150 peut translater le long de l'axe principal (alpha) de l'ouverture 113 du guide 111, le set de réglage secondaire 150 peut pivoter autour de la barre rigide 110 et finalement la barre de réglage final 130 peut pivoter autour de l'axe de révolution (Bêta) du premier guide de perçage 132. Le chirurgien utilise ces réglages pour obtenir la mise en position souhaitée de la barre de réglage finale 130 comme expliquée précédemment et serre les mollettes 92, 91, 112 pour bloquer les réglages.

Le genou est amené en dessous du premier guide de perçage 132 et la broche filetée 80 est introduite dans le genou G du patient le long de l'axe de révolution (Bêta) grâce au porte broche 181 guidé par sa portée cylindrique 180 le long de l'orifice interne 135b et perpendiculairement au plan défini par la table d'opération 100 (Figure 39).

Le choix du site d'insertion de la broche filetée 180 est tout à fait aléatoire, mais une fois la localisation choisie, il faut impérativement garder " la mémoire " de cette localisation.

Le chirurgien pivote le membre inférieur du patient dans le plan frontal et amène le genou G sous le second guide 131, la barre de réglage final 130 étant toujours libre de pivoter autour de l'axe (Bêta) du premier guide de perçage 132, ce qui permet d'ajuster aisément sa position dans l'espace (Figure 39).

L'ajustement est correct lorsque la portée cylindrique 182 du porte-broche 81 " retrouve " l'axe de la broche filetée précédemment introduite dans le genou G, ce qui permet à ce moment de l'extraire du genou G.

Avant le retrait du porte broche 181, il est impératif de serrer la molette 133 de manière à conserver la "mémoire" de la localisation de la broche en position 2 du genou (=F2). La localisation dans l'espace du deuxième point appartenant à l'arc de cercle décrit par la broche lorsque le genou est en position P2 (=F2) est ainsi déterminée. Il est bien compris que le système de barres articulées est désormais complètement rigide par rapport à la table d'opération.

Le bloc de visée 160 est alors mis en place le long de la barre de réglage final 130 et centré avec le téton de localisation 134. La tige d'alignement 168 peut également être mise en place dans la seconde fente 164 du bloc de visée 160

Par construction, le plan de symétrie (Omega) de la seconde fente 164 qui inclut l'axe de révolution du tenon cylindrique 163 du bloc de visée 160 et celui de l'axe de la barre d'alignement 168 indique l'orientation de la perpendiculaire passant par le milieu exact du segment qui sépare les axes de révolution (Bêta, Gamma) des guides de perçages 131, 132, indiquant ainsi de façon extrêmement précise la direction du centre de la tête fémorale (O).

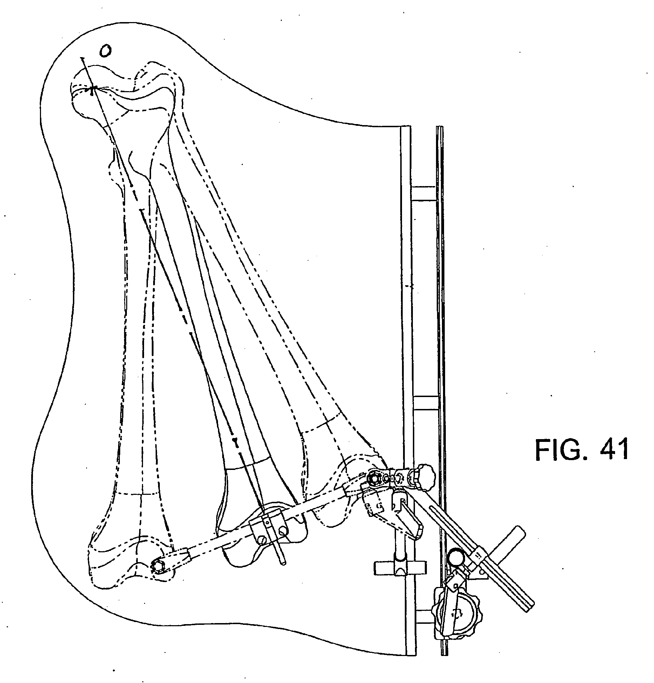

Le centre du genou G est alors amené à l'aplomb vertical du tenon cylindrique 163 ou de la barre d'alignement 168 et on a ainsi matérialisé l'axe mécanique fémoral (3x) (Figure 41).

On peut alors fixer deux broches 182, 183 au travers des orifices internes 161, 162 du guide de visée 160. Par construction, les deux broches 161, 162 définissent un plan perpendiculaire par rapport à l'axe mécanique fémoral (3x).

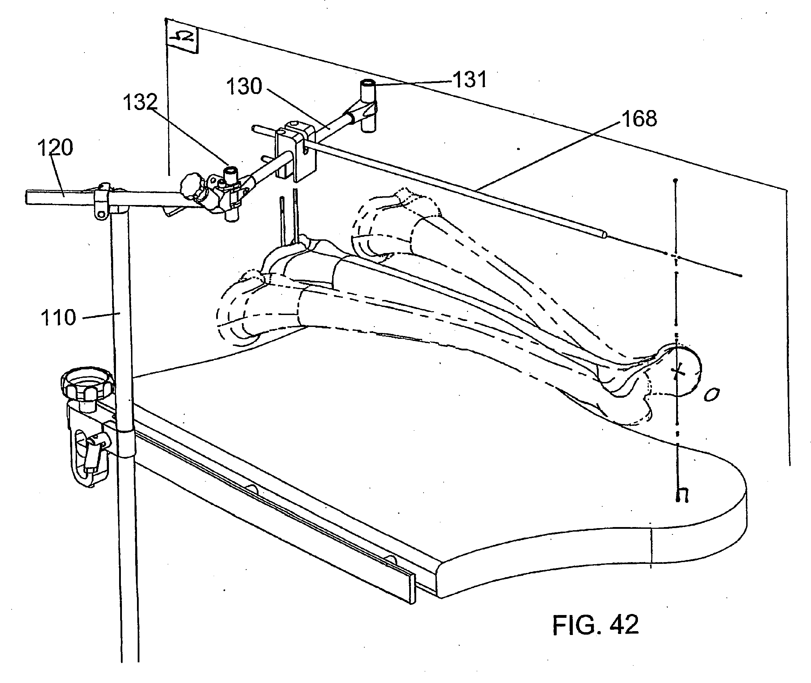

Par la figure 42, il est illustré que le centre de la tête fémorale (O) appartient au plan de symétrie (Omega) de la seconde fente 64.

Si désiré, le chirurgien peut desserrer la molette 171 du renvoi articulaire 170. Le dévissage de la vis de blocage 71 autour de son axe de rotation (Téta) permet de pivoter le renvoi angulaire 170 et la barre de réglage final 130 par rapport à la barre de réglage 120 et de ce fait de basculer le plan défini par les axes de révolution (Bêta, Gamma) de perpendiculaire au plan frontal du patient à perpendiculaire au plan sagittal du patient, ce qui lui permet de déterminer le centre de la tête fémorale dans le plan sagittal selon une méthode similaire à celle expliqué pour la détermination du centre de la tête fémorale dans le plan frontal.

Après détermination du centre de la tête fémorale (O) dans le plan frontal ( et donc détermination de l'axe mécanique (3x1)) et potentiellement détermination du centre de la tête fémorale (O) dans le plan sagittal, le chirurgien peut desserrer la molette 91 de l'étau 90 et retirer verticalement vers le haut le set de barres 110, 120, 130 ainsi que le guide de visée 160 et la barre d'alignement 168.

Les instruments de coupe distale fémorale orientés par les broches mises lors de la détermination du centre de la tête fémorale (O) permettront ainsi d'obtenir une coupe distale rigoureusement perpendiculaire à l'axe mécanique fémoral.

La figure 43 divulgue un autre mode de réalisation s'affranchissant de la nécessité d'attacher un étau 90 au rail 101 de la table d'opération 100. Cet autre mode de réalisation se présente sous la forme d'un étau de maintien 210 muni de pinces latérales 211, 212 qui viennent s'attacher au bassin ou à la cage thoracique du patient. Par l'action d'un mécanisme connu (non représenté) tel qu'une vis sans fin, les pinces latérales 211, 212 peuvent se serrer (c'est à dire se déplacer l'une vers l'autre) et venir se bloquer contre le patient. Ainsi l'étau de maintien 210 est entièrement solidaire du patient. Parce que l'étau de maintien 210 dispose d'une large platine 214 posée au contact de la table d'opération et que la barre rigide 110 est perpendiculaire par rapport à cette platine 114, la barre rigide 110 est donc verticale. Le système de barre articulées 110, 120, 130 est similaire à la version précédente. Le système de réglage des barres 110, 120, 130 entre elles est également similaire à la version précédente, à la seule différence que le guide 111 peut également se translater verticalement le long de la barre rigide 110.

Il est également possible de définir une solution hybride (non représentée) constituée d'étaux latéraux s'attachant de part et d'autre de la table venant enserrer le patient.

La fig. 44 illustre schématiquement le principe pour un autre mode de réalisation, faisant appel à un montage en pantographe consistant en quatre bras articulés disposés en losange dont deux sommets 51, 52 opposés constituent des axes dans lesquels peut coulisser librement la tige de visée 53. Une alternative pour l'arrangement en pantographe est illustrée à la fig. 26. Un des sommets 58 du montage en losange est fixé en pivotement à un bras 57 lui même fixé, éventuellement en pivotement, à un élément de la table d'opération 55. Une tige 53 reliant deux sommets opposés passera toujours perpendiculairement au milieu du segment de droite (diagonale) reliant les deux autres sommets (58, 59), bloqués après repérage en F1 et F2, du quadrilatère.

Le blocage de l'axe au niveau de F1 suffit à figer l'orientation de la tige 53.

Selon encore une autre variante dont le principe est illustré à la fig. 45, on peut prévoir une barre 60, sous la forme d'un bras, de préférence une barre graduée, perpendiculaire ou non à l'axe 55 de la table d'opération, ce bras étant fixe ou pivotant en 64. Ce bras comprend un premier moyen de repérage 61. Le second moyen de repérage 62 est fixé sur une barre télescopique 63 pouvant se rétracter au moins partiellement et être immobilisée dans le bras 60. Un élément 65, par exemple du type curseur, avec le dispositif de visée peut coulisser sur la barre 63 et être amené au centre des deux moyens de repérage 61, 62, eux-mêmes immobilisés après la détection des points F1 et F2 du même point du genou en position P1 et P2.

## Revendications

1. Appareil pour la détermination d'un plan contenant l'axe mécanique du fémur comprenant un dispositif dans lequel on prévoit:
- un moyen mécanique d'éléments articulés et/ou coulissants (3, 140, 150) pour la détermination et la mémorisation de deux positions dans l'espace (F1 et F2) d'un même point quelconque (F) du genou lorsque celui-ci se trouve en position P1 et P2, par rapport à un référentiel configuré pour être fixe par rapport au bassin du patient, les dites positions étant obtenues par pivotement du membre inférieur depuis la position P1 à la position P2, autour du centre de la tête fémorale
- un moyen mécanique pour matérialiser l'orientation d'un plan oméga passant par le milieu du segment de droite défini par les deux dites positions F1 et F2, perpendiculairement à ce segment et contenant le centre de la tête fémorale.

2. Appareil selon la revendication 1 dans lequel , ledit moyen mécanique d'éléments articulés et/ou coulissants (3, 140, 150) comporte un élément (4) portant les deux moyens de mémorisation ( en 30 ,30') de deux positions dans l'espace et destiné à être immobilisé par rapport audit référentiel.

3. Appareil selon la revendication 1 ou 2 dans lequel on prévoit pour la mémorisation des points F1 et F2 au moins un moyen d'immobilisation mécanique d'une barre articulée (4, 130) supportée par un dispositif à au moins trois degrés de liberté et comportant au moins deux moyens de repérage (30, 131, 132) dudit point F quelconque du genou.

4. Appareil selon la revendication 3 dans lequel le dispositif comprend un degré de liberté de rotation autour d'un axe perpendiculaire au plan sagittal ou frontal

5. Appareil selon la revendication 3 ou 4 dans lequel les moyens de repérage sont des éléments cylindriques creux (30) aptes à se positionner avec précision au dessus d'un repère fixé au point F du genou, repère obtenu par exemple par l'action d'un faisceau laser, d'un crayon ou marqueur chirurgical ou d'un bistouri électrique.

6. Appareil selon n'importe laquelle des revendications 2 à 5 dans lequel les moyens de repérage comprennent des guides de perçage (30, 131, 132) ou de marquage.

7. Appareil selon la revendication précédente dans lequel le repère est une broche (35, 180) éventuellement filetée.

8. Appareil selon la revendication 2 dans lequel le moyen pour matérialiser l'orientation du plan oméga est un moyen mécanique permettant d'orienter un élément de direction (12, 168) au milieu et perpendiculairement au segment de droite séparant les deux moyens de repérage (5, 6, 131, 132).

9. Appareil selon la revendication précédente dans lequel on prévoit en outre un moyen permettant de reporter et fixer sur le fémur l'élément de direction dudit centre de la tête du fémur.

10. Appareil selon la revendication précédente dans lequel l'élément de direction est une tige (12, 168).

11. Appareil selon la revendication précédente dans lequel la tige comprend un moyen de fixation au fémur et est apte à être désolidarisée du reste de l'appareil.

12. Appareil selon n'importe laquelle des revendications précédentes dans lequel ledit référentiel est une table d'opération.

13. Appareil selon n'importe laquelle des revendications précédentes dans lequel ledit référentiel est le bassin associé audit fémur.

14. Appareil selon la revendication 13 comprenant un moyen de fixation (210) audit bassin, par exemple au niveau de l'os iliaque, par exemple au niveau de la crête iliaque.

15. Appareil selon n'importe laquelle des revendications précédentes dans lequel le moyen mécanique d'éléments articulés comprend quatre tiges articulées formant un parallélogramme dont deux sommets opposés (58, 59) sont munis des moyens de repérage, un des sommets (58) étant solidarisé audit référentiel, et les deux autres sommets (51, 52) étant associés au moyen pour déterminer l'orientation du plan oméga.

16. Appareil selon n'importe laquelle des revendications 1, 3 à 14 dans lequel un des moyens de repérage (62) est situé sur un bras coulissant (63) dans ou le long d'un autre bras (60) fixé audit référentiel et comprenant l'autre moyen de repérage(61).

17. Appareil selon n'importe laquelle des revendications 1 à 16 **caractérisé en ce qu'**il comprend en plus un ensemble de réglage par éléments articulés (140) apte à faire pivoter d'un angle de 90° ledit moyen mécanique d'éléments articulés ou coulissants (150) de sorte que la barre de réglage final peut passer d'un plan frontal à un plan sagittal.

## Claims

1. An instrument for the determination of a plane containing the mechanical axis of the femur of an inferior limb comprising a device in which there is provided:
- a mechanical means made of articulated and /or sliding elements in order to determine and memorize two positions, freely selected, in space (F1 and F2) of the same point (F) of the knee, when said knee is brought in positions P1 and P2, relative to a referential system configured in order to be fixed relative to the patient pelvis, positions being obtained by rotation of the inferior limb from the position P1 towards the position P2, about the centre of the femoral head,
- a mechanical means for materializing an orientation of a plane omega passing through the middle of a line segment defined by the two said positions F1 and F2, perpendicular to this segment and containing the centre of the femoral head.

2. Instrument according to claim 1 wherein the said mechanical means of articulated and/or sliding elements (3, 140, 150) comprises an element (4) supporting both memorization means (30, 30') of two positions in space and destined to be immobilized relative to set referential.

3. Instrument according to claim 1 or 2 wherein there is provided for the memorization of points F1 and F2 at least one means of mechanical immobilization of an articulated bar (4,130) supported by a device with at least three degrees of liberty and comprising at least two localization means (30, 131, 132) of said freely selected point F of the knee.

4. Instrument according to claim 3 wherein the device comprises a degree of rotation about a perpendicular axis to the sagittal or frontal plane.

5. Instrument according to claim 3 or 4 wherein the localization means are hollow cylindrical elements (30) able to be precisely positioned above a fixed mark located at point F of the knee, said mark being provided by the action for example of a laser beam, a pen or surgical marker or an electrical lancet.

6. Instrument according to any of the claims 2 to 5 wherein the localization means comprise drilling (30, 131, 132) or marking guides.

7. Instrument according to the preceding claim wherein the mark is a pin (35, 180), possibly threaded.

8. Instrument according to claim 2 wherein the means for materializing the orientation of the omega plane is a mechanical means allowing the orientation of a direction element (12, 168) at the middle and perpendicular to the straight line segment separating the two localization means (5, 6, 131, 132).

9. Instrument according to the previous claim wherein there is furthermore provided a means allowing to carry and fix the direction element of the aforementioned centre of the femoral head on the femur.

10. Instrument according to the preceding claim wherein the direction element is a rod (12, 168).

11. Instrument according to the previous claims wherein the rod is fixed to the femur and can be disconnected from the remainder of the device.

12. Instrument according to any of the preceding claims wherein the said referential system is a surgery table.

13. Instrument according to any of the preceding claims wherein the said referential system is the pelvis associated to said femur.

14. Instrument according to claim 13 comprising a fixation means to the pelvis, for example at the level of the iliac bone, for example at the level of the iliac cavity.

15. Instrument according to any of the preceding claims wherein the mechanical means of articulated elements comprises four articulated rods forming a parallelogram whose two opposite angles (58, 59) are provided with localization means, one (58) of the angles being attached to the said referential system, and the two other angles (51, 52) being associated to the means for determining the omega plane's orientation.

16. Instrument according to any of the claims 1, 3 to 14 wherein one of the localization means (62) is located on a sliding arm (63) in or along another arm (60) fixed to the said referential system and comprising the other localization means (61).

17. Instrument according to any of the claims 1 to 16 **characterised in that** it comprises furthermore an adjustment set by articulated elements (140) being able to make rotate around an angle of 90° said mechanical means of articulated or sliding elements, so that the final adjustment bar can pass from a frontal plane to a sagittal plane.

## Patentansprüche

1. Instrument zur Bestimmung einer die mechanische Achse des Oberschenkelknochens enthaltenden Ebene, wobei das besagte Instrument
- ein mechanisches aus gelenkigen und / oder verschiebbaren Elementen (3, 140, 150) bestehendes Mittel zur Bestimmung und zur Speicherung von zwei Positionen im Bereich (F1 und F2) von irgendeinem selben Punkt (F) des Knies, wenn sich dieser in Position F1 und F2 relativ zu einem Bezugssystem befindet, das so konfiguriert ist, dass es in Bezug auf das Becken des Patienten ortsfest ist, wobei die genannten Positionen erreicht werden, indem das untere Glied von der Position F1 in die Position F2, um die Mitte des Oberschenkelkopfs geschwenkt wird,
- ein mechanisches Mittel zur Ausrichtung einer Omega-Ebene, die durch die Mitte des durch die beiden genannten Positionen F1 und F 2 definierten rechten Segments senkrecht zu diesem Segment verläuft und in der die Mitte des Oberschenkelkopfs enthalten ist,
umfasst.

2. Instrument nach Anspruch 1, bei dem das genannte mechanische aus gelenkigen und / oder verschiebbaren Elementen (3, 140, 150) bestehende Mittel ein Element (4) umfasst, dass die beiden Mittel (in 30, 3o') zur Speicherung von zwei räumlichen Positionen trägt und dazu bestimmt ist, gegenüber dem genannten Bezugssystem fixiert zu werden.

3. Instrument nach Anspruch 1 oder 2, bei dem zur Speicherung der Punkte F1 und F2 mindestens ein Mittel vorgesehen ist, das dazu bestimmt ist, eine gelenkige Stange (4, 130), die von einer Vorrichtung mit mindestens drei Freiheitsgraden getragen wird, mechanisch zu fixieren und mindestens zwei Mittel (30, 131, 132) zur Lokalisierung des genannten irgendeinen Punkts (F) des Knies umfasst.

4. Instrument nach Anspruch 1, bei dem die Vorrichtung einen Freiheitsgrad für die Drehung um eine senkrecht zur sagitallen oder frontalen Ebene stehende Achse umfasst.

5. Instrument nach Anspruch 1, bei dem die Lokalisierungsmittel aus hohlen, zylindrischen Elementen (30) bestehen, die so ausgebildet sind, dass sie sich mit Genauigkeit oberhalb eines auf dem Punkt (F) des Knies fixierten Merkzeichen positionieren, wobei das genannte Merkzeichen zum Beispiel mit Hilfe eines Laserstrahls, eines Bleistifts oder eines chirurgisches Markers oder eines elektrischen Messers angebracht ist.

6. Instrument nach irgendeinem der vorangehenden Ansprüche 2 bis 5, bei dem die Lokalisierungsmittel Führungen für Bohrungen oder Markierung (30, 131, 132) umfassen.

7. Instrument nach dem vorangehenden Anspruch, bei dem das Merkzeichen aus einem Stab (35, 180) besteht, der eventuell mit einem Gewinde versehen ist.

8. Instrument nach Anspruch 2, bei dem das Mittel zur Ausrichtung der Omega - Ebene aus einem mechanischen Mittel besteht, das die Möglichkeit bietet, ein Richtelement (12, 168) mittig und senkrecht zu dem die beiden Lokalisierungsmittel (5, 6, 131, 132) trennenden rechten Segment auszurichten.

9. Instrument nach dem vorangehenden Anspruch, bei dem ein zusätzliches Mittel vorgesehen ist, mit dem das Richtelement der genannten Mitte des Oberschenkelkopfs auf dem Oberschenkelkochen angebracht und fixiert werden kann.

10. Instrument nach dem vorangehenden Anspruch, bei dem das Richtelement aus einer Stange (12, 168) besteht.

11. Instrument nach dem vorangehenden Anspruch, bei dem die Stange ein Mittel zur Befestigung an den Oberschenkelknochen umfasst und von dem übrigen Instrument getrennt werden kann.

12. Instrument nach irgendeinem der vorangehenden Ansprüche, bei dem das genannte Bezugssystem ein Operationstisch ist.

13. Instrument nach irgendeinem der vorangehenden Ansprüche, bei dem das genannte Bezugssystem das dem Oberschenkelknochen zugehörige Becken ist.

14. Instrument nach Anspruch 13, das ein Mittel (210) umfasst, das zur Befestigung an das Becken, z. B. auf der Ebene des iliakalen Knochen, z. B. auf der Ebene des Beckenkamms vorgesehen ist.

15. Instrument nach irgendeinem der vorangehenden Ansprüche, bei dem das genannte mechanische aus gelenkigen oder verschiebbaren Elementen (150) bestehende Mittel vier gelenkige Stangen umfasst, die ein Parallelogramm bilden, dessen zwei entgegen gesetzte Spitzen (58, 59) mit Merkzeichen versehen sind, wobei eine der Spitzen (58) dem Bezugssystem zugeordnet ist, und die zwei anderen Spitzen (51, 52) dem Mittel zur Bestimmung der Ausrichtung der Omega - Ebene zugeordnet sind.

16. Instrument nach irgendeinem der vorangehenden Ansprüche 1, 3 bis 14, bei dem das eine (62) der Lokalisierungsmittel auf einem Arm (63) angeordnet ist, der entlang eines anderen Armes oder in diesem anderen Arm (60) verschiebbar ist, wobei der genannte andere Arm auf dem Bezugssystem fixiert ist und das andere Lokalisierungsmittel (61) umfasst.

17. Instrument nach irgendeinem der vorangehenden Ansprüche 1 bis 16, **dadurch gekennzeichnet, dass** es ferner ein Verstellaggregat umfasst, mit gelenkigen Elementen (140), die das genannte mechanische aus gelenkigen oder verschiebbaren Elementen (150) bestehende Mittel um einen Winkel von 90 ° so schwenken können, dass die Stange von einer frontalen Ebene zu einer sagittalen Ebene bei der Endverstellung versetzt werden kann.
